# EUROPÄISCHE PATENTSCHRIFT

(11) **EP 1 597 319 B1**
(45) Veröffentlichungstag und Bekanntmachung des Hinweises auf die Patenterteilung: **04.04.2007**
(21) Anmeldenummer: 04715272.3
(22) Anmeldetag: 27.02.2004
(51) Int. Cl.: C09B 5/62, C07H 21/00

(54) **MONOFUNKTIONALISIERTE PERYLENTETRACARBONSÄUREBISIMIDE**
MONOFUNCTIONALIZED PERYLENETETRACARBOXYLIC ACID BISIMIDES
BISIMIDES D'ACIDE PERYLENE TETRACARBOXYLIQUE MONOFONCTIONNALISES

(30) Priorität: 28.02.2003 DE 10308941
(43) Veröffentlichungstag der Anmeldung: 23.11.2005
(73) Patentinhaber: Max-Planck-Gesellschaft zur Förderung der Wissenschaften e.V., 80539 München (DE)
(72) Erfinder: KOHL, Christopher, 55127 Mainz (DE); QU, Jianqiang, 68169 Mannheim (DE); MÜLLEN, Klaus, 50939 Köln (DE)
(74) Vertreter: Weiss, Wolfgang
(86) Internationale Anmeldenummer: PCT/EP2004/001971
(87) Internationale Veröffentlichungsnummer: WO 2004/076563

(56) Entgegenhaltungen:
- WO-A-01/69254
- WO-A-99/31069
- QUANTE H ET AL: "NOVEL PERYLENE-CONTAINING POLYMERS" MACROMOLECULAR CHEMISTRY AND PHYSICS, WILEY VCH, WEINHEIM, DE, Bd. 197, November 1996 (1996-11), Seiten 4029-4044, XP008017838 ISSN: 1022-1352 in der Anmeldung erwähnt

## Beschreibung

Die Erfindung betrifft neue Perylentetracarbonsäurebisimid-Derivate mit verbesserten Anwendungseigenschaften.

Perylentetracarbonsäurebisimide sind für ihre außergewöhnliche thermische, chemische und fotophysikalische Stabilität bekannt (1). Sie werden als Farbstoffe und Pigmente, beispielsweise in reprografischen Prozessen (2), fluoreszierenden Solarkollektoren (3), fotovoltaischen Zellen (4) und Farbstofflasern (5) eingesetzt. Ein weiteres mögliches Anwendungsgebiet ist der Einsatz als Markierungsgruppen in Nachweisverfahren, insbesondere in diagnostischen oder analytischen Verfahren an biologischen Proben, einschließlich lebender Zellen. Viele dieser Anwendungen basieren auf der hohen Fluoreszenzintensität der Perylen-Chromophor-Gruppe und darauf, dass die Fluoreszenzanregungs-Emissionswellenlängen von Perylentetracarbonsäurebisimiden bei Wellenlängen von oberhalb 500 nm liegen, bei denen Signalstörungen, verursacht durch Autofluoreszenz von Zellen, biologischen Geweben oder biologischen Flüssigkeiten, vernachlässigbar sind.

Ein Nachteil bekannter Perylentetracarbonsäurebisimide besteht jedoch darin, dass sie eine schlechte Wasserlöslichkeit und/oder eine schwache Fluoreszenzintensität in Wasser aufweisen (6). Diese Nachteile werden hauptsächlich durch die Aggregation von Farbstoffmolekülen in einer hydrophilen Umgebung bewirkt, wodurch die Anzahl biologischer Anwendungen begrenzt ist (7).

In DE-A-37 03 513 werden Perylentetracarbonsäurebisimide beschrieben, die einen oder mehrere Sulfonsäurereste in der Imidstruktur aufweisen.

Quante et al. (Macromol. Chem. Phys. 197 (1996), 4029-4044) offenbaren Perylentetracarbonsäurebisimide, die Sulfonsäuregruppen am Grundgerüst des Perylen-Chromophors enthalten. Weitere modifizierte Perylentetracarbonsäurebisimide werden in EP-A-0 648 817, EP-A-0 654 506, US-A-4,378,302, EP-A-0 869 959, WO 97/22607 sowie von Zhubanov et al. (Zh. Org. Khim. 28 (1992), 1486-1488) beschrieben.

Aus EP 0 896 964 sind Perylenhydrazidimide bekannt, die als Nachweisreagenz für Carbonylverbindungen verwendet werden können.

WO 02/14414 offenbart funktionalisierte Perylentetracarbonsäurebisimide, die als Initiatoren oder/und als Reaktionspartner für Polymerisationsreaktionen bereitgestellt werden.

WO 01/69254 offenbart Perylentetracarbonsäurebisimide, die eine Gruppe zur kapplung an einen Bindepartner enthalten.

Diese Verbindungen zeigen eine erhöhte Fluoreszenz in wässrigen Lösungen. Doch die Nachteile des Standes der Technik, insbesondere die Neigung zur Aggregatbildung in wässrigen Lösungen, konnten nicht vollständig beseitigt werden.

Es besteht daher ein großes Bedürfnis, neue Perylentetracarbonsäurebisimide mit verbesserten Eigenschaften, insbesondere hinsichtlich der Fähigkeit zur Kopplung an Bindepartner, der Wasserlöslichkeit oder/und der Fluoreszenzintensität in Wasser oder wässrigen Medien, bereitzustellen.

Diese Aufgabe wird erfindungsgemäß dadurch gelöst, dass monofunktionalisierte Perylentetracarbonsäurebisimide bereitgestellt werden, die vorzugsweise mindestens zwei hydrophile Gruppen am Grundgerüst des Perylen-Chromophors aufweisen. Diese zeigen Fluoreszenzquantenausbeuten von bis zu 80 % in Wasser und können definiert an Bindepartner, z.B. Biomoleküle, gekoppelt werden.

Ein Gegenstand der Erfindung sind somit Perylentetracarbonsäurebisimide der Strukturformel (I) 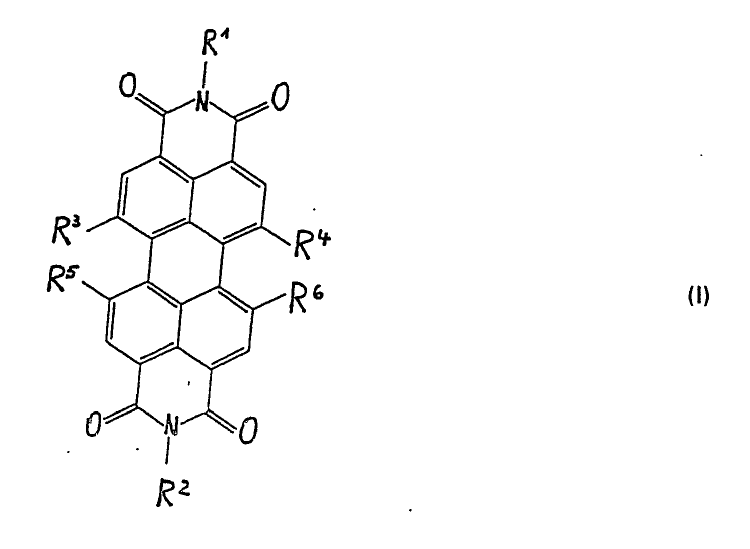 worin R¹ und R² unterschiedliche organische Reste bedeuten, wobei einer der Reste eine Gruppe zur Kopplung an einen Bindepartner aufweist, und mindestens zwei von R³, R⁴, R⁵ und R⁶ jeweils unabhängig organische Reste bedeuten, die mindestens eine hydrophile Gruppe enthalten.

Ein wesentliches Merkmal der Erfindung besteht darin, dass die Verbindungen (I) monofunktionalisierte Verbindungen sind, d.h. Verbindungen, bei denen einer der beiden Reste R¹ und R² eine Gruppe zur Kopplung an einen Bindepartner aufweist. Diese Kopplungsgruppen sind vorzugsweise reaktive Funktionalitäten, die eine Kopplung an Bindepartner, insbesondere an Amino-, Thiol- oder/und Carboxylgruppen in biologischen Substanzen ermöglichen, oder Vorstufen von solchen reaktiven Funktionalitäten. Beispiele für geeignete biologische Substanzen sind Nukleobasen, Nukleoside, Nukleotide und Analoga davon, z.B.

Nukleotidderivate zur chemischen Synthese von Nukleinsäuren, wie Phorsphoramidite, Nukleinsäuren, wie DNA oder RNA, oder auch Nukleinsäureanaloga, wie etwa PNA oder LNA, Aminosäuren, Aminosäurederivate, Peptide, Polypeptide, Glycoproteine, Mono-, Oligo- und Polysaccharide, Lipide etc.

Beispiele geeigneter reaktiver Funktionalitäten sind Aktivester, Maleimide Isocyanate, Sulfonylhalogenide, Carbonsäurehalogenide, insbesondere Carbonsäurechloride, Jodacetamide, Aziridine, Epoxide, Acylazide und Acylnitrile. Beispiele von Vorstufen für reaktive Funktionalitäten sind Sulfonsäuregruppen und Carbonsäuregruppen, die nach bekannten Methoden in reaktive Funktionalitäten überführt werden können. Diese Überführung erfolgt vorzugsweise nach der Synthese des Verbindungsgrundgerüsts, wobei gegebenenfalls zusätzlich Schutzgruppen zur Vermeidung unerwünschter Nebenreaktionen, z.B. an den Positionen R³, R⁴, R⁵ und R⁶, verwendet werden können.

Die als Bestandteil der lmidstruktur vorliegenden Reste R¹ und R² sind vorzugsweise über ein sekundäres oder tertiäres Kohlensstoffatom an das Imid-Stickstoffatom gebunden, wobei jedoch auch eine Bindung über ein primäres Kohlenstoffatom, d.h. über eine CH₂-Gruppe möglich ist. Besonders bevorzugt sind R¹ und R² sekundäre oder tertiäre aliphatische Reste oder cyclische Reste mit üblicherweise 3-30 Kohlenstoffatomen, insbesondere mono- oder bicyclische aromatische oder heteroaromatische Reste, wie etwa Phenyl, Pyridyl oder Naphthyl, die gegebenenfalls einen oder mehrere Substituenten tragen. Beispiele für geeignete Substituenten für aliphatische oder gesättigte cyclische Reste sind CN, NO₂, Halogen (z.B. F, Cl, Br oder I), OH, OR⁷, OCOR⁷, SH, SR⁷, SCOR⁷, SO₂R⁷, CHO, COR⁷, COOH, COOM, COOR⁷, CONH₂, CONHR⁷, CON(R⁷)₂, SO₃H, SO₃M, SO₃R⁷, NH₂, NHR⁷ oder N(R⁷)₂, wobei M ein Kation, z.B. ein Alkalimetallion, wie Natrium, Kalium etc., ist und R⁷ einen gegebenenfalls Halogen-substituierten C₁-C₆-Alkylrest darstellt. Cyclische Reste, z.B. aromatische oder heteroaromatische Reste können zusätzlich durch einen oder mehrere Reste R⁷ substituiert sein.

Mindestens zwei der Reste R³, R⁴, R⁵ und R⁶ tragen mindestens eine hydrophile Gruppe. Vorzugsweise tragen alle vier Reste R³, R⁴, R⁵ und R⁶ mindestens eine hydrophile Gruppe. Die hydrophile Gruppe kann eine ungeladene oder geladene Gruppe sein. Beispiele für geeignete ungeladene Gruppen sind Hydroxylgruppen und Poly-Oxy-C₂-C₄-Alkylengruppen, insbesondere Polyoxyethylengruppen, mit 3 oder mehr, z.B. bis zu 50 oder 100 Alkylenoxideinheiten. Bevorzugt ist die hydrophile Gruppe jedoch eine geladene Gruppe, d.h. eine in neutralen Medien, z.B. bei pH 7, geladene Gruppe, beispielsweise eine positiv geladene Gruppe, wie etwa eine Aminogruppe oder eine Ammoniumgruppe, insbesondere eine quaternisierte Ammoniumgruppe, oder ein alkyliertes heteroaromatisches N-Atom, insbesondere eine N-Alkyl-Pyridinium-, N-Alkyl-Chinolinium- oder N-Alkyl-Isochinoliniumgruppe, wobei der Alkylrest vorzugsweise bis zu 6 Kohlenstoffatome aufweist und gegebenenfalls wie zuvor beschrieben substituiert sein kann. Beispiele für geeignete negativ geladene Gruppen sind Sulfonsäure- bzw. Carbonsäuregruppen, SO₃H und COOH sowie deren Salze SO₃M und COOM, wobei M ein Kation, z.B. ein Alkalimetallion, wie etwa Kalium oder Natrium, bedeutet. Darüber hinaus können R³, R⁴, R⁵ oder/und R⁶ auch mehrere gleich oder entgegengesetzt geladene Gruppen enthalten, wobei im letzteren Fall amphiphile Gruppen entstehen. Besonders bevorzugte amphiphile Gruppen sind heteroaromatische N-Atome, die mit einem Rest alkyliert sind, der eine -CO₂H, -SO₃H, -CO₂M oder -SO₃M-Gruppe trägt.

Weiterhin ist bevorzugt, dass zumindest zwei der Reste R³, R⁴, R⁵ und R⁶ aromatische oder heteroaromatische Reste, insbesondere monocyclische oder bicyclische Reste, wie etwa Phenyl oder Pyridin, umfassen.

In einer besonders bevorzugten Ausführungsform werden mindestens zwei von R³, R⁴, R⁵ und R⁶ durch die allgemeine Strukturformel (II)

-O-Ar (II)

repräsentiert, worin Ar einen aromatischen oder heteroaromatischen Rest bedeutet, der mindestens eine hydrophile, z.B. eine geladene Gruppe wie zuvor angegeben, enthält. Im Falle, dass Perylentetracarbonsäurebisimide mit negativen Ladungsträgern verwendet werden, kann mindestens einer von R³, R⁴, R⁵ und R⁶ die allgemeine Strukturformel (III) aufweisen: 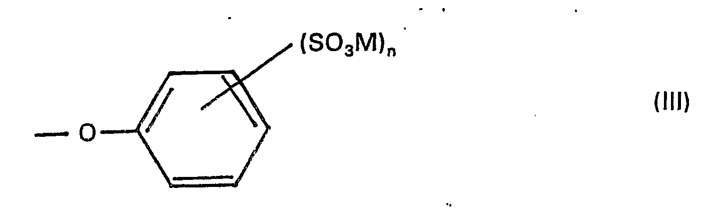 worin M ein Kation ist und n 1, 2 oder 3 ist.

Im Falle der Verwendung von Perylentetracarbonsäurebisimiden mit positiven Ladungsträgern kann mindestens einer von R³, R⁴, R⁵ und R⁶ die allgemeine Strukturformel (IV) aufweisen: 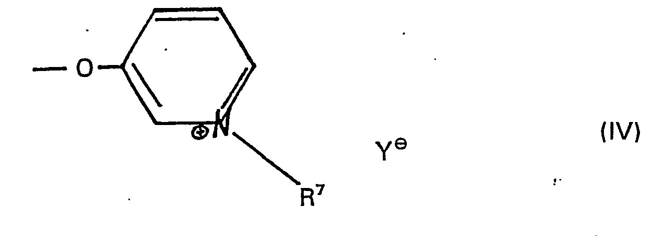 worin R⁷ einen gegebenenfalls substituierten C₁-C₆-, vorzugsweise C₁-C₄-Alkylrest darstellt und Y ein Anion, z.B. ein Halogenidion, ist. Wenn der Rest R⁷ als Substituent eine negativ geladene Gruppe trägt, erhält man einen amphiphilen Rest.

Die erfindungsgemäßen Perylentetracarbonsäurebisimide werden üblicherweise aus einem technisch gut zugänglichen Di- oder Tetrahalogensubstituierten Perylentetracarbonsäurebisanhydrid der allgemeinen Strukturformel (V) 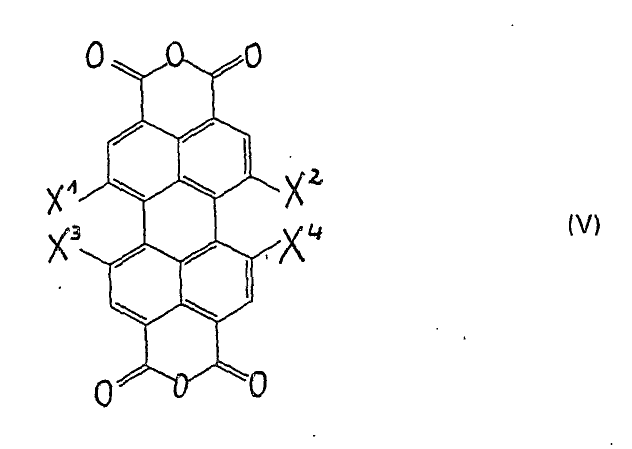 worin mindestens zwei von X¹, X², X³ und X⁴ Halogen, insbesondere Cl oder Br, und die übrigen Wasserstoff bedeuten, durch Kondensation mit zwei unterschiedlichen primären Aminen, H₂NR¹ und H₂NR², worin R¹ und R² wie zuvor definiert sind, hergestellt. Besonders bevorzugte molare Verhältnisse der Amine sind 1 bis 3 Äquivalente Amin bezogen auf 1 Äquivalent di- oder tetrahalogensubstituiertes Perylentetracarbonsäurebisanhydrid, wobei das sterisch anspruchsvollere Amin im höheren Überschuss eingesetzt wird. Überraschenderweise wurde festgestellt, dass bei dieser Reaktion ein gemischt substituiertes Produkt in hoher Ausbeute anfällt, das von eventuell auftretenden Nebenprodukten ohne Weiteres, z.b. säulenchromatographisch, abgetrennt werden kann. Die resultierenden Halogen-substituierten Perylentetracarbonsäurebisimide der allgemeinen Strukturformel (VI) 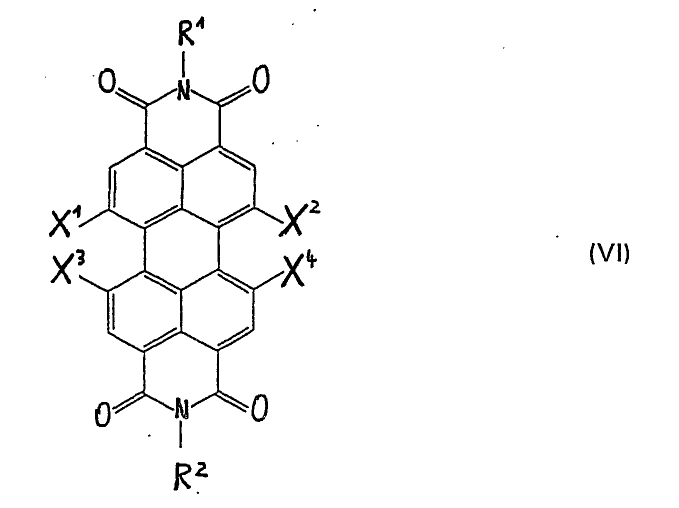 worin mindestens zwei von X¹, X², X³ und X⁴ Halogen, insbesondere Cl oder Br, und die übrigen Wasserstoff bedeuten, werden anschließend mit einer Verbindung der allgemeinen Strukturformel (VII)

HO-Ar' (VII)

umgesetzt, worin Ar' einen aromatischen oder heteroaromatischen Rest bedeutet. Bei dieser Reaktion entstehen tetra-substituierte Perylentetracarbonsäurebisimide der allgemeinen Strukturformel (VIIIa) oder disubstituierte Perylentetracarbonsäurebisimide der allgemeinen Strukturformel (VIIIb):

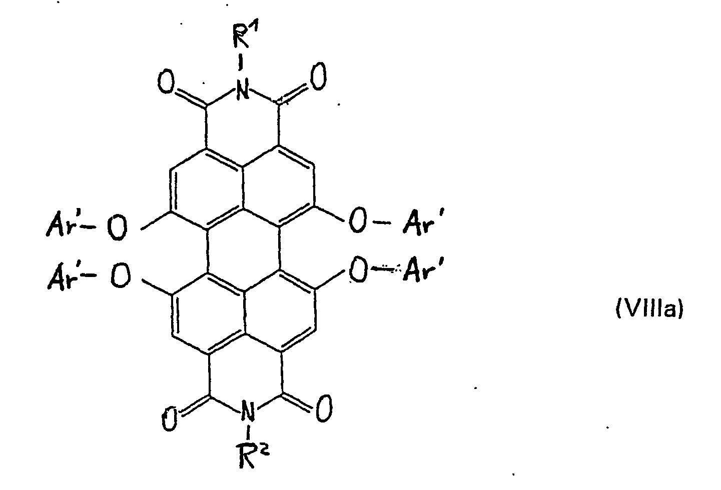

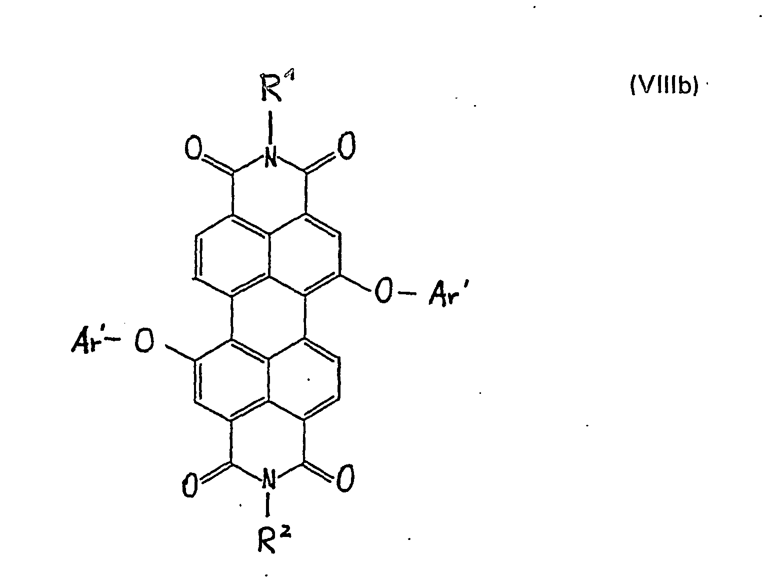

Anschließend können hydrophile Gruppen, wie zuvor angegeben, in die aromatischen oder heteroaromatischen Reste Ar' eingeführt werden. Beispielsweise kann die Einführung von mindestens einer Gruppe SO₃H oder SO₃M in Ar' durch Umsetzung mit H₂SO₄ unter geeigneten Bedingungen erfolgen. Andererseits kann eine Alkylierung von heteroaromatischen N-Atomen in Ar' erfolgen, z.B. durch Umsetzung mit einem Alkylhalogenid, z.B. CH₃l, unter geeigneten Bedingungen.

Nach Synthese des Verbindungsgrundgerüsts kann eine Überführung der Vorstufe einer reaktiven Funktionalität in R¹ oder R² in die reaktive Funktionalität selbst erfolgen.

Die monofunktionalisierten Verbindungen (I) können anschließend, gegebenenfalls nach Entfernung von Schutzgruppen, kovalent mit einem Bindepartner, z.B. einem Biomolekül wie zuvor angegeben, gekoppelt werden. Die Erfindung betrifft somit auch Konjugate der Verbindungen (I) mit einem Bindepartner.

Die erfindungsgemäßen Verbindungen können weiterhin in allen für den Einsatz von Perylentetracarbonsäurebisimiden geeigneten technischen Gebieten angewendet werden, beispielsweise in Farbstofflasern, als Markierungsgruppen in analytischen Verfahren, als Tracer, in Szintillationszählern, in Fluoreszenz-Solarkollektoren, in Flüssigkristallen, in Kaltlichtquellen, bei der Materialprüfung, als Fotoleiter, in fotografischen Verfahren, in Beleuchtungs- und Anzeigelementen, als Halbleiter etc.

Die Verbindungen oder deren Konjugate, z.B. kovalente Konjugate mit Biomolekülen, wie Nukleinsäuren, Proteinen, Peptiden, Sacchariden etc., können in Flüssigkeiten, z.B. organischen oder/und wässrigen Lösungsmitteln, oder in Festkörpern, z.B. Kunststoffen, gelöst werden.

Weiterhin soll die vorliegende Erfindung durch Abbildungen und Beispiele näher erläutert werden.

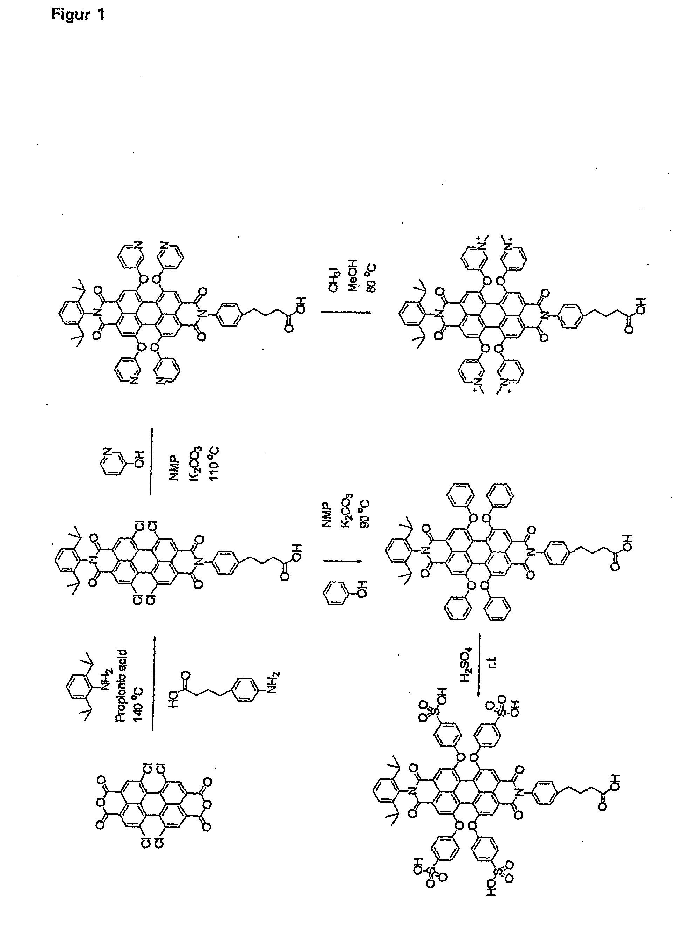

**Figur 1** zeigt ein Reaktionsschema zur Herstellung der in den Beispielen 1-5 genannten tetra-substituierten Verbindungen.

### Beispiele

### Beispiel 1: HerstellungvonN-(2,6-diisopropylphenyl)-N'-[(4-buttersäure)-phenyl]-1,6,7,12-tetrachlorperylen-3,4:9,10-tetracarbonsäurebisimid

Ein Gemisch von 13,25 g (25 mmol) Tetrachlorperylen-3,4:9,10-tetracarbonsäurebisanhydrid, 13,5 g Diisopropylanilin und 5 g (28 mmol) Buttersäureanilin in 400 ml Propionsäure wurden in einem Schlenkkolben unter Argon auf 140 °C erhitzt und bei dieser Temperatur für 12 h belassen. Nach Abkühlen auf Raumtemperatur wurde das Reaktionsgemisch in Wasser gegossen und das Rohprodukt durch Filtration gesammelt. Das gewünschte N,N'-unterschiedlich substituierte Produkt wurde durch Säulenchromatographie an Kieselgel mit CH₂Cl₂/Aceton (20: 1) als Eluent aufgereinigt.

M.P.: > 300 °C; ¹H-NMR (250 MHz, CD₂Cl₂, 300 K): δ[ppm]:8,76 (s, 2 H), 8,74 (s, 2 H), 7,54 (t, 1 H), 7,45 (d, 2 H), 7,38 (d, 2 H), 7,28 (d, 2 H), 2,80 (m, 4 H), 2,73 (m, 2 H); 2,09 (m, 2 H), 1,16 (d, 12 H). UV-Vis Spektrum (Chloroform) λₘₐₓ (∈) = 527 (46791), 494 (32499), 436 nm (12120 M⁻¹ cm⁻¹) Fluoreszenz-Spektrum (Chloroform) λₘₐₓ = 553 nm. FD Massenspektrum (8 kV):m/z = 849,7 (100 %) [M⁺] (Cal. 850,5)

### Beispiel 2: Herstellung von N-(2,6-diisopropylphenyl)-N'-[(4-buttersäure)-phenyl]-1 ,6,7, 12-tetraphenoxyperylen-3,4:9, 1 0-tetracarbonsäurebisimid

8,48 g (10 mmol) der in Beispiel 1 hergestellten Verbindung, 5,4 g (100 mmol) Phenol und 8,62 g (68,5 mmol) K₂CO₃ wurden unter einer Inertgasatmosphäre in 400 mmol N-Methylpyrrolidon (NMP) suspendiert. Das Reaktionsgemisch wurde auf 90 °C erhitzt und für 24 h gerührt. Nach Abkühlen auf Raumtemperatur wurde das Gemisch in 5 I wässrige HCl gegossen. Das resultierende Präzipitat wurde filtriert, neutral gewaschen und bei 75 °C unter Vakuum getrocknet. Das Produkt wurde weiter durch Chromatographie an Kieselgel mit CH₂Cl₂/Aceton (10:1) als Eluent aufgereinigt.

M.P.: > 300 °C; ¹H-NMR (250 MHz, CD₂Cl₂, 300 K):δ [ppm]:8,18 (s, 2 H), 8,17 (s, 2 H), 7,46 (t, 1 H), 7,31 (m, 12 H), 7,15 (m, 6 H), 7,00 (d, 8 H); 2,72 (m, 4 H), 2,42 (m, 2 H), 2,01 (m 2 H), 1,09 (d, 12 H). UV-Vis Spektrum (Chloroform) λₘₐₓ = 573, 546 nm; Fluoreszenz-Spektrum (Chloroform) ^{λ}max = 605 nm. FD Massenspektrum (8 kV):m/z = 1081,8 (100 %) [M⁺] (Cal. 1081,1)

### Beispiel 3: Herstellung von N-(2,6-diisopropylphenyl)N'-[(4-buttersäure)-phenyl] -1,6,7, 12-tetra(4-sulfonylphenoxy)-perylen-3,4:9,10-tetracarbonsäurebisimid

0,5 g (0,46 mmol) der in Beispiel 2 hergestellten Verbindung wurden in 1 ml konzentrierter Schwefelsäure gelöst und für 16 h bei Raumtemperatur (20 °C) gerührt. Das Produkt wurde durch Zugabe von Wasser präzipitiert, filtriert und in Vakuum bei 75 °C getrocknet.

M.P.: >300 °C; ¹H-NMR (250 MHz, MeOD, 300 K):δ[ppm]:8,01 (s, 2 H), 7,99 (s, 2 H), 7,69 (d, 4 H), 7,65 (d, 2 H), 7,25 (t, 1 H), 7,12 (m, 6 H), 6,95 (m, 8 H), 2,56 (m, 4 H), 2,20 (m, 2 H); 1,82 (m, 2 H), 0,94 (d, 12 H). UV-Vis Spektrum (Wasser) λₘₐₓ = 575, 546 nm; Fluoreszenz-Spektrum (Wasser) λₘₐₓ = 607 nm; Maldi-Tof Massenspektrum m/z = 1402,1 (100 %) [M⁺] (Cal. 1401,4).

### Beispiel4: HerstellungvonN-(2,6-diisopropylphenyl)-N'-[(4-buttersäure)-phenyl]-1,6,7,12-tetra(3-pyridoxy)perylen-3,4:9,10-tetracarbonsäurebisimid

1,5 g (1,77 mmol) der in Beispiel 1 hergestellten Verbindung, 1,1 g (10,7 mmol) 3-Hydroxypyridin und 1,2 g (0,8 mmol) K₂CO₃ wurden in 150 ml NMP gelöst. Das Reaktionsgemisch wurde bei 110 °C unter einer Inertgasatmosphäre gerührt. Nach 36 h wurde auf Raumtemperatur abgekühlt und mit verdünnter Salzsäure neutralisiert. Das Rohprodukt wurde filtriert, mit Wasser gewaschen und bei 75 °C in Vakuum getrocknet. Der erhaltene Feststoff wurde durch Chromatographie an Kieselgel mit CH₂Cl₂/ Aceton (10:1) als Eluent weiter aufgereinigt.

M.P.: > 300 °C; ¹H-NMR (250 MHz, CD₂Cl₂, 300 K): δ[ppm]: 8,33 (m, 8 H), 8,23 (s, 2 H), 8,19 (s, 2 H), 7,46 (t, 1 H), 7,36-7,15 (m, 14 H), 2,71 (m, 4 H), 2,38 (m, 2 H), 1,98 (m, 2 H), 1,08 (d, 12 H). UV-Vis Spektrum (Chloroform) λₘₐₓ(∈) = 564 (50150), 528 (32536), 446 nm (16523 M⁻¹ cm⁻¹); Fluoreszenz-Spektrum (Chloroform) λₘₐₓ = 595 nm; FD Massenspektrum (8 kV): m/z = 1086,2 (100 %) [M⁺] (Cal. 1085,1).

### Beispiel 5: HerstellungvonN-(2,6-diisopropylphenyl)-N'-[(4-buttersäure)-phenyl]-1,6,7,12-tetra[3-(N-methylpyridinium)oxy]perylen-3,4:9,10-tetracarbonsäurebisimid

300 mg (0,28 mmol) der in Beispiel 4 hergestellten Verbindung wurde bei 80 °C in 100 ml Methanol gelöst. Zu der gerührten Lösung wurden 2 ml Methyljodid gegeben und das Gemisch für 48 h unter Rückfluss gehalten. Das resultierende Produkt fiel in hoher Reinheit an.

M.P.: >300 °C; ¹H-NMR (250 MHz, MeOD, 300 K): δ[ppm]: 9,52-9,29 (m, 4 H), 9,06 (m, 4 H), 8,83-8,77 (m, 8 H), 8,38 (m, 4 H), 7,80 (t, 1 H), 7,70 (m, 4 H), 7,60 (d, 2 H), 5,27 (s, 12 H), 3,10 (m, 4 H), 2,75 (m, 2 H), 2,36 (m, 2 H), 1,45 (d, 12 H). UV-Vis Spektrum (Wasser) λₘₐₓ(ε) = 547 (29974), 525 (23496), 432 nm (9456 M⁻¹ cm⁻¹); Fluoreszenz-Spektrum (Wasser) λₘₐₓ = 591 nm.

### Beispiel 6: Herstellung von disubstituierten Perylentetracarbonsäurebisimiden

Ausgehend von N,N'-Bis(2,6-diisopropylphenyl)-1,7-dibromperylen-3,4:9,10-tetracarbonsäurebisimid wurden, entsprechend der in den Beispielen 1 bis 3 beschriebenen Reaktionsführung, N-(2,6-Diisopropylphenyl)-N'-[(4-buttersäure)-phenyl]-1,7-di(4-sulfonylphenoxy)perylen-3,4:9,10-tetra- carbonsäurebisimid und, entsprechend der in den Beispielen 1 und 4-5 beschriebenen Reaktionsführung, N-(2,6-Diisopropylphenyl)-N'-[(4-buttersäure)-phenyl]-1,7-di-[3-(N-methylpyridinium)oxy]perylen-3,4:9,10-tetracarbonsäurebisimid hergestellt.

### Referenzen:

(1) Y. Nagao und T. Misono, Dyes Pigm., 1984, 5, 171
   A. Rademacher, S. Merkle und H. Lanhals, Chem. Ber. 1982, 115, 2927
(2) H.O. Loutfy, A.M: Hor, P. Kazmaier and M. Tam, J. lmaging Sci., 1989, 33, 151
(3) G. Seybold und G. Wagenblast, Dyes Pigm. 1989, 11, 303
(4) L. Schmidt-Mende, A. Fechtenkötter, K. Müllen, E. Moons, R.H. Friend, J.D. MacKenzie, Science, 2001, 293, 1119
(5) R. Gvishi, R. Reisfeld und Z. Bursheim, Cehm. Phys. Lett., 1993, 213,338
(6) H. Icil, D. Uzun und N. Pasaogullari, Spectrosc. Lett., 1998, 31, 667 S. lcil, S. Demic, B. Dindar, A.O. Doroshenko und C. Timur, J. Photochem. Photobiol., 2000, 136, 15
   H. Quante, P. Schlichting, U. Rohr, Y. Geerts und K. Müllen, Macromol. Chem. Phys., 1996, 197, 4029
   W. Bauer, D. Baumgart, D. Schnaltmann, K.-P. Kreutzer und W. Zöller, EP 0 832 937 B1
   H.-A. Klok, J. Rodriguez Hernandez, S. Becker und K. Müllen, J. Polym. Sci., 2001, 39, 1572
(7) H. Han, R.J. Bennett und L.H. Hurley, Biochem. 2000, 39, 9311 N.V. Khromov-Borisov, M.L. lndenbom und A.F. Danilov, Pharm. Chem. J. 1980, 14, 90

## Patentansprüche

1. Perylentetracarbonsäurebisimide der Strukturformel (I) 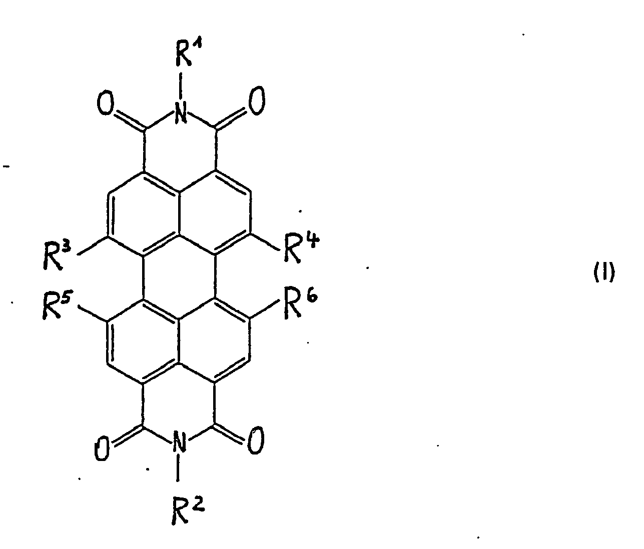 worin R¹ und R² unterschiedliche organische Reste bedeuten, wobei einer der Reste eine Gruppe zur Kopplung an einen Bindepartner aufweist, und
mindestens zwei von R³, R⁴, R⁵ und R⁶ jeweils unabhängig organische Reste bedeuten, die mindestens eine hydrophile Gruppe enthalten.

2. Verbindungen nach Anspruch 1,
**dadurch gekennzeichnet,**
**dass** die Kopplungsgruppe eine reaktive Funktionalität zur Bindung an Amino-, Thiol- oder Carboxylgruppen oder eine Vorstufe davon ist.

3. Verbindungen nach Anspruch 1 oder 2,
**dadurch gekennzeichnet,**
**dass** die Kopplungsgruppe ein Aktivester, Maleimid, lsocyanat, Sulfonylhalogenid, Carbonsäurehalogenid, Jodacetamid, Aziridin, Epoxid, Acylazid, Acylnitril oder eine Vorstufe davon ist.

4. Verbindungen nach einem der Ansprüche 1 bis 3,
**dadurch gekennzeichnet,**
**dass** R¹ oder/und R² sekundäre oder tertiäre aliphatische Reste oder cyclische Reste darstellen.

5. Verbindungen nach einem der Ansprüche 1 bis 4,
**dadurch gekennzeichnet,**
**dass** R¹ oder/und R² aromatische oder heteroaromatische Reste, insbesondere Phenyl-, Pyridyl- oder Naphthylreste darstellen, die gegebenenfalls einen oder mehrere Substituenten enthalten.

6. Verbindungen nach einem der Ansprüche 1 bis 5,
**dadurch gekennzeichnet,**
**dass** mindestens zwei von R³, R⁴, R⁵ und R⁶ einen organischen Rest bedeuten, der eine ungeladene hydrophile Gruppe trägt.

7. Verbindungen nach einem der Ansprüche 1 bis 6,
**dadurch gekennzeichnet,**
**dass** mindestens zwei von R³, R⁴, R⁵ und R⁶ jeweils unabhängig einen organischen Rest bedeuten, der eine (in neutralen Medien) positiv geladene Gruppe trägt.

8. Verbindungen nach einem der Ansprüche 1 bis 7,
**dadurch gekennzeichnet,**
**dass** mindestens zwei von R³, R⁴, R⁵ und R⁶ jeweils unabhängig einen organischen Rest bedeuten, der eine Gruppe ausgewählt aus quaternären Ammonium- und N-alkylierten heteroaromatischen N-Gruppen, wie N-Alkyl-Pyridinium-, N-Alkyl-Chinolinium- oder N-Alkyl-Isochinolinium-Gruppen, trägt.

9. Verbindungen nach einem der Ansprüche 1 bis 8,
**dadurch gekennzeichnet,**
**dass** R³, R⁴, R⁵ und R⁶ jeweils unabhängig einen organischen Rest bedeuten, der eine (in neutralen Medien) negativ geladene Gruppe trägt.

10. Verbindungen nach einem der Ansprüche 1 bis 9,
**dadurch gekennzeichnet,**
**dass** mindestens zwei von R³, R⁴, R⁵ und R⁶ jeweils unabhängig einen organischen Rest bedeuten, der eine Gruppe ausgewählt aus SO₃H, COOH, SO₃M und COOM trägt, wobei M ein Kation bedeutet.

11. Verbindungen nach einem der Ansprüche 1 oder 10,
**dadurch gekennzeichnet,**
**dass** R³, R⁴, R⁵ oder/und R⁶ aromatische oder heteroaromatische Reste, insbesondere Phenyl- oder Pyridinreste umfassen.

12. Verbindungen nach einem der Ansprüche 1 bis 11,
**dadurch gekennzeichnet,**
**dass** mindestens einer von R³, R⁴, R⁶ und R⁶ die allgemeine Strukturformel (II) aufweist:
-O-Ar (II)
worin Ar einen aromatischen oder heteroaromatischen Rest bedeutet, der mindestens eine geladene Gruppe enthält.

13. Verbindungen nach Anspruch 12,
**dadurch gekennzeichnet,**
**dass** mindestens einer von R³, R⁴, R⁵ und R⁶ die allgemeine Strukturformel (III) aufweist: 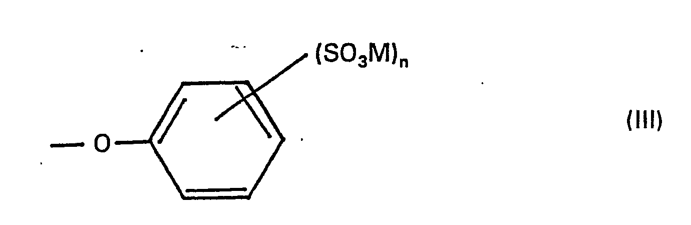 worin M ein Kation ist und n eine ganze Zahl von 1-3 ist.

14. Verbindungen nach Anspruch 13,
**dadurch gekennzeichnet,**
**dass** mindestens einer von R³, R⁴, R⁵ und R⁶ die allgemeine Strukturformel (IV) aufweist: 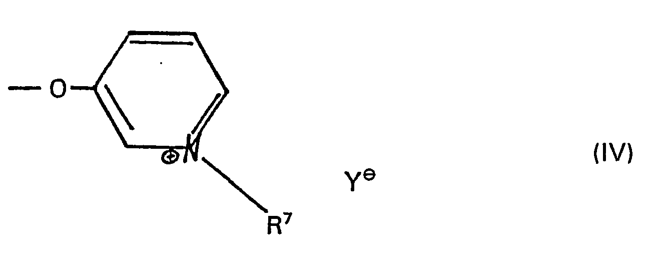 worin R⁷ einen gegebenenfalls substituierten C₁-C₆-Alkylrest darstellt und Y ein Anion ist.

15. Konjugate von Verbindungen nach einem der Ansprüche 1 bis 14 mit einem Bindepartner.

16. Konjugate nach Anspruch 15,
**dadurch gekennzeichnet,**
**dass** der Bindepartner ein Biomolekül ist.

17. Verfahren zur Herstellung von Perylentetracarbonsäurebisimiden der allgemeinen Strukturformel (I), umfassend die Schritte:
(a) Umsetzen von Halogen-substituierten Perylendi- oder Perylentetracarbonsäureanhydriden der allgemeinen Strukturformel (V): 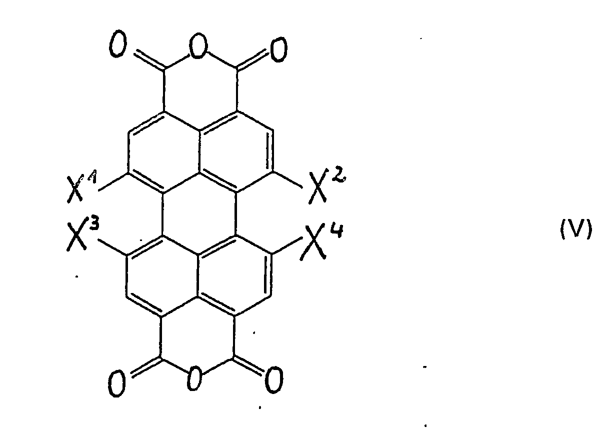 worin mindestens zwei von X¹, X², X³ und X⁴ Halogen, insbesondere Cl oder Br, und die anderen Wasserstoff bedeuten, mit einem Gemisch von zwei unterschiedlichen Aminen H₂NR¹ und H₂NR², worin R¹ und R² wie in Anspruch 1 definiert sind, zu einer Verbindung der allgemeinen Strukturformel (VI) 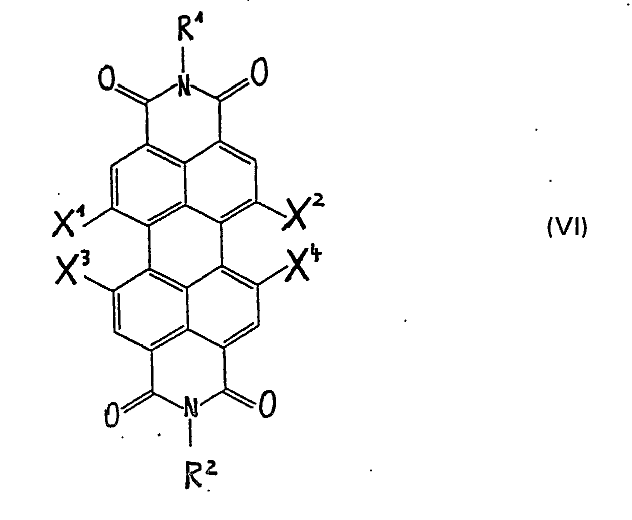
(b) Umsetzen der Verbindung (VI) mit einer Verbindung der allgemeinen Strukturformel (VII):
HO - Ar' (VII)
worin Ar' einen aromatischen oder heteroaromatischen Rest bedeutet,
zu tetra-substituierten Perylentetracarbonsäurebisimiden der allgemeinen Strukturformel (Vllla) oder zu disubstituierten Perylentetracarbonsäurebisimiden der allgemeinen Strukturformel (VIIIb): 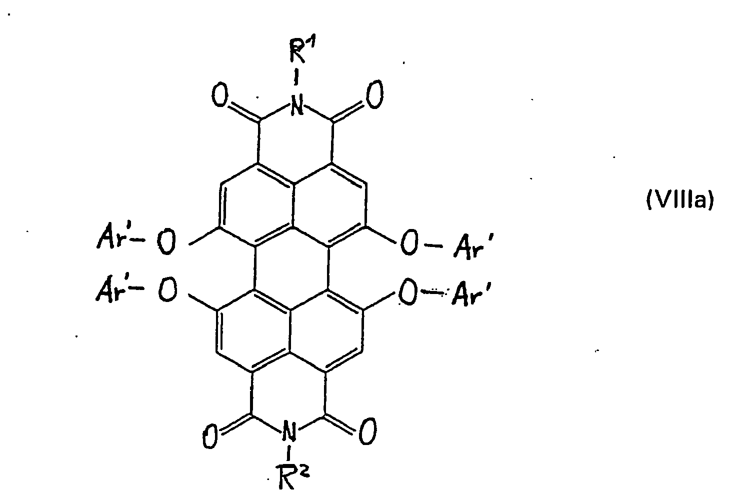 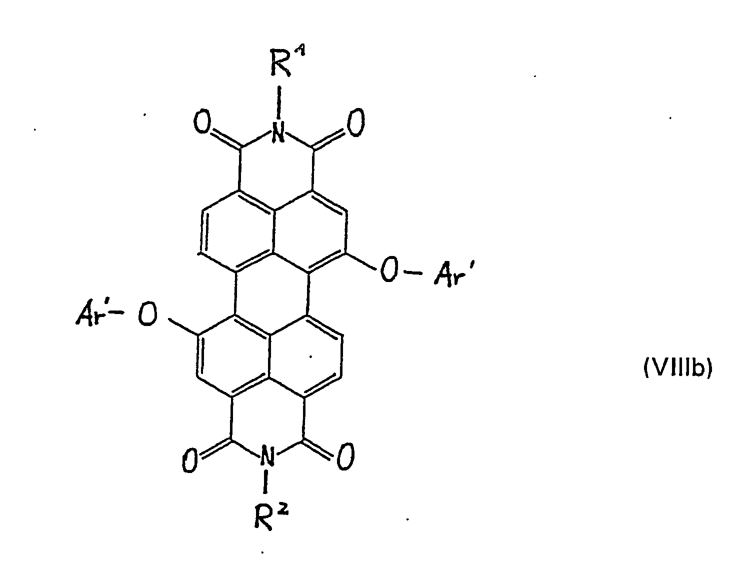 und
(c) Einführen von hydrophilen Gruppen in die aromatischen oder heteroaromatischen Reste Ar'.

18. Verfahren nach Anspruch 17,
**dadurch gekennzeichnet,**
**dass** Schritt (b) die Einführung von mindestens einer Gruppe SO₃H oder SO₃M in Ar' umfasst, wobei M ein Kation ist.

19. Verfahren nach Anspruch 17,
**dadurch gekennzeichnet,**
**dass** Schritt (b) die Alkylierung mindestens eines heteroaromatischen N-Atoms in Ar' umfasst.

## Claims

1. A perylenetetracarboxylic acid bisimide of the structural formula (I) 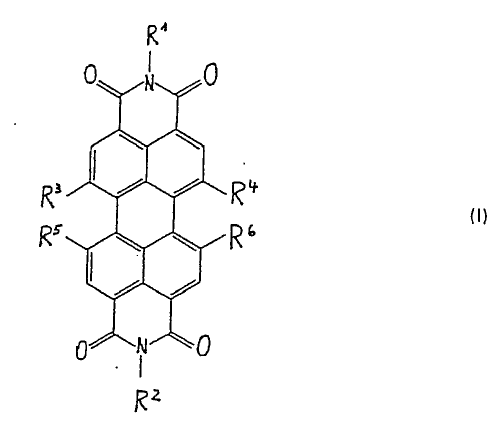 wherein R¹ and R² are different organic radicals, one of the radicals having a group for coupling to a binding partner, and
at least two of R³, R⁴, R⁵ and R⁶ are each independently organic radicals which contain at least one hydrophilic group.

2. A compound as claimed in claim 1,
**characterized in that**
the coupling group is a reactive functionality for binding to amino, thiol or carboxyl groups or a precursor thereof.

3. A compound as claimed in claim 1 or 2,
**characterized in that**
the coupling group is an active ester, maleimide, isocyanate, sulfonyl halide, carbonyl halide, iodoacetamide, aziridine, epoxide, acyl azide, acyl nitrile or a precursor thereof.

4. A compound as claimed in any of claims 1 to 3,
**characterized in that**
R¹ or/and R² are secondary or tertiary aliphatic radicals or cyclic radicals.

5. A compound as claimed in any of claims 1 to 4,
**characterized in that**
R¹ or/and R² are aromatic or heteroaromatic radicals, in particular phenyl, pyridyl or naphthyl radicals, which optionally contain one or more substituents.

6. A compound as claimed in any of claims 1 to 5,
**characterized in that**
at least two of R³, R⁴, R⁵ and R⁶ are an organic radical which bears an uncharged hydrophilic group.

7. A compound as claimed in any of claims 1 to 6,
**characterized in that**
at least two of R³, R⁴, R⁵ and R⁶ are each independently an organic radical which bears a positively charged (in neutral media) group.

8. A compound as claimed in any of claims 1 to 7,
**characterized in that**
at least two of R³, R⁴, R⁵ and R⁶ are each independently an organic radical which bears a group selected from quaternary ammonium and N-alkylated heteroaromatic N groups such as N-alkylpyridinium, N-alkylquinolinium or N-alkylisoquinolinium groups.

9. A compound as claimed in any of claims 1 to 8,
**characterized in that**
R³, R⁴, R⁵ and R⁶ are each independently an organic radical which bears a negatively charged (in neutral media) group.

10. A compound as claimed in any of claims 1 to 9,
**characterized in that**
at least two of R³, R⁴, R⁵ and R⁶ are each independently an organic radical which bears a group selected from SO₃H, COOH, SO₃M and COOM, wherein M is a cation.

11. A compound as claimed in any of claims 1 to 10,
**characterized in that**
R³, R⁴, R⁵ or/and R⁶ comprise aromatic or heteroaromatic radicals, in particular phenyl or pyridine radicals.

12. A compound as claimed in any of claims 1 to 11,
**characterized in that**
at least one of R³, R⁴, R⁵ and R⁶ has the general structural formula (II):
-O-Ar (II)
wherein Ar is an aromatic or heteroaromatic radical which contains at least one charged group.

13. A compound as claimed in claim 12,
**characterized in that**
at least one of R³, R⁴, R⁵ and R⁶ has the general structural formula (III): 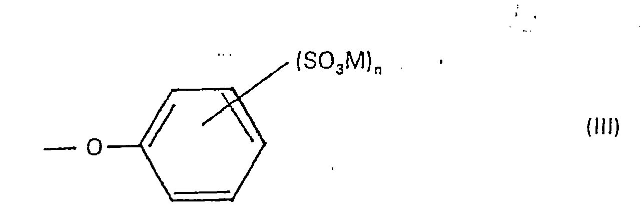 wherein M is a cation and n is an integer of 1-3.

14. A compound as claimed in claim 13,
**characterized in that**
at least one of R³, R⁴, R⁵ and R⁶ has the general structural formula (IV): 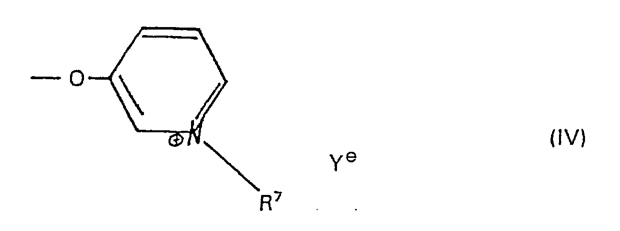 wherein R⁷ is an optionally substituted C₁-C₆-alkyl radical and Y is an anion.

15. A conjugate of compounds as claimed in one of claims 1 to 14 with a binding partner.

16. The conjugate as claimed in claim 15,
**characterized in that**
the binding partner is a biomolecule.

17. A process for preparing perylenetetracarboxylic acid bisimides of the general structural formula (I), comprising the steps of:
(a) reacting halogen-substituted perylenedi- or perylenetetracarboxylic anhydrides of the general structural formula (V): 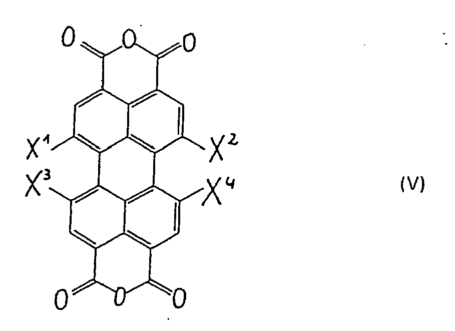 wherein at least two of X¹, X², X³ and X⁴ are halogen, in particular Cl or Br, and the others are hydrogen
with a mixture of two different amines H₂NR¹ and H₂NR², wherein R¹ and R² are each as defined in claim 1, to give a compound of the general structural formula (VI) 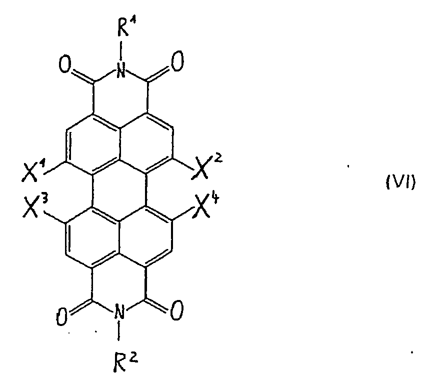
(b) reacting the compound (VI) with a compound of the general structural formula (VII):
HO - Ar' (VII)
wherein Ar' is an aromatic or heteroaromatic radical
to give tetra-substituted perylenetetracarboxylic acid bisimides of the general structural formula (VIIIa) or to give disubstituted perylenetetracarboxylic acid bisimides of the general structural formula (VIIIb): 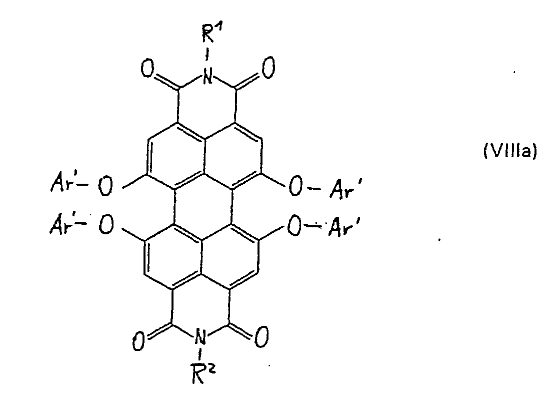 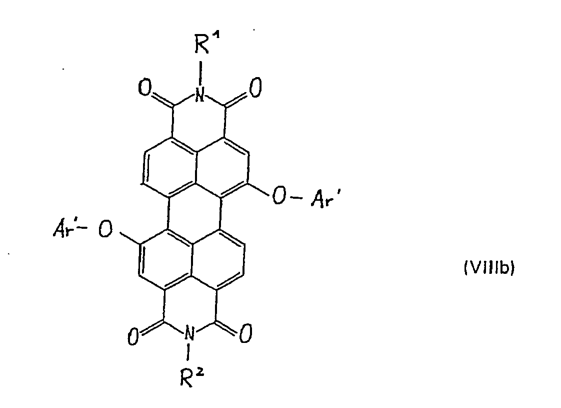 and
(c) introducing hydrophilic groups into the aromatic or heteroaromatic Ar' radicals.

18. The process as claimed in claim 17,
**characterized in that**
step (b) includes the introduction of at least one SO₃H or SO₃M group into Ar', where M is a cation.

19. The process as claimed in claim 17,
**characterized in that**
step (b) includes the alkylation of at least one heteroaromatic N atom in Ar'.

## Revendications

1. Bisimides de l'acide pérylène tétracarboxylique de formule structurale (I) 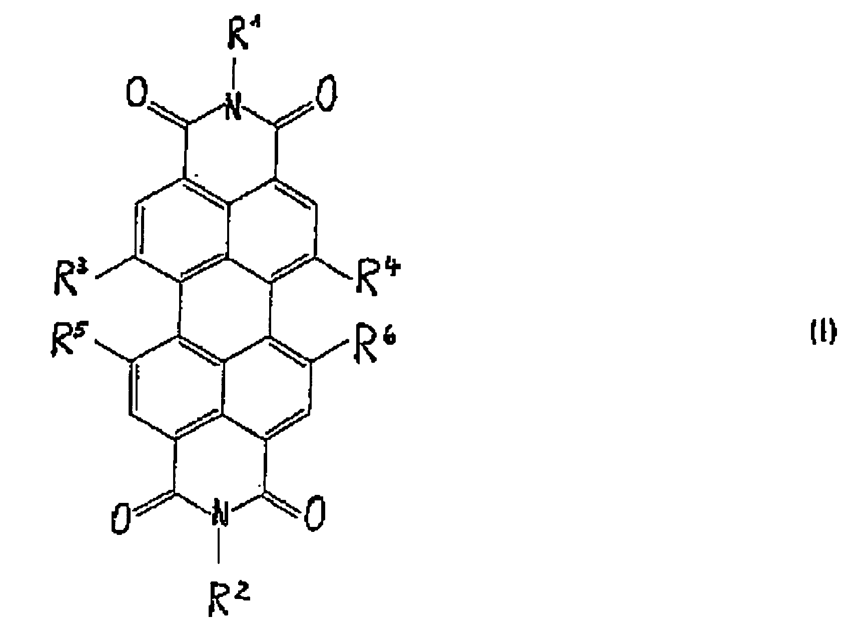 où R¹ et R² représentent des restes organiques différents, dans lesquels un des restes présente un groupe pour le couplage à un partenaire de liaison, et
au moins deux groupes parmi R³, R⁴, R⁵ et R⁶, chacun indépendamment, représentent des restes organiques qui contiennent au moins un groupe hydrophile.

2. Composés selon la revendication 1, **caractérisés en ce que** le groupe de couplage est un groupe fonctionnel réactif pour la liaison à des groupes amino, thiol ou carboxy ou son précurseur.

3. Composés selon la revendication 1 ou la revendication 2, **caractérisés en ce que** le groupe de couplage est un ester actif, un maléimide, un isocyanate, un halogénure de sulfonyle, un halogénure d'acide carboxylique, un acétamide d'iode, un aziridine, un époxyde, un azide d'acyle, un nitrile d'acyle ou leur précurseur.

4. Composés selon l'une quelconque des revendications 1 à 3, **caractérisés en ce que** R¹ ou/et R² représentent un reste aliphatique secondaire ou tertiaire ou un reste cyclique.

5. Composés selon l'une quelconque des revendications 1 à 4, **caractérisés en ce que** R¹ ou/et R² représentent des restes aromatiques ou hétéroaromatiques, en particulier des restes phényle, pyridyle ou naphtyle, qui le cas échéant contiennent un ou plusieurs substituants.

6. Composés selon l'une quelconque des revendications 1 à 5, **caractérisés en ce qu'**au moins deux groupes parmi R³, R⁴, R⁵ et R⁶ représentent un reste organique qui porte un groupe hydrophile non chargé.

7. Composés selon l'une quelconque des revendications 1 à 6, **caractérisés en ce qu'**au moins deux groupes parmi R³, R⁴, R⁵ et R⁶, chacun indépendamment, représentent un reste organique qui porte (dans un milieu neutre) un groupe chargé positivement.

8. Composés selon l'une quelconque des revendications 1 à 7, **caractérisés en ce qu'**au moins deux groupes parmi R³, R⁴, R⁵ et R⁶ chacun indépendamment, représentent un reste organique qui porte un groupe choisi parmi les groupes ammonium quaternaires et les groupes N- hétéroaromatiques N-alkylés, comme les groupes N-alkyl-pyridinium, N-alkyl-chinolinium ou N-alkyl-isochinolinium.

9. Composés selon l'une quelconque des revendications 1 à 8, **caractérisés en ce que** R³, R⁴, R⁵ et R⁶, chacun indépendamment, représentent un reste organique qui porte (dans un milieu neutre) un groupe chargé négativement.

10. Composés selon l'une quelconque des revendications 1 à 9, **caractérisés en ce qu'**au moins deux groupes parmi R³, R⁴, R⁵ et R⁶, chacun indépendamment, représentent un reste organique qui porte un groupe choisi parmi SO₃H, COOH, SO₃M et COOM, sachant que M représente un cation.

11. Composés selon l'une quelconque des revendications 1 à 10, **caractérisés en ce que** R³, R⁴, R⁵ ou/et R⁶ comprennent des restes aromatiques ou hétéroaromatiques, en particulier les restes phényle ou pyridine.

12. Composés selon l'une quelconque des revendications 1 à 11, **caractérisés en ce qu'**au moins un groupe parmi R³, R⁴, R⁵ et R⁶ présente la formule structurale générale (II):
-O-Ar (II)
dans laquelle Ar représente un reste aromatique ou hétéroaromatique qui contient au moins un groupe chargé.

13. Composés selon la revendication 12, **caractérisés en ce qu'**au moins un groupe parmi R³, R⁴, R⁵ et R⁶ présente la formule structurale générale (III) : 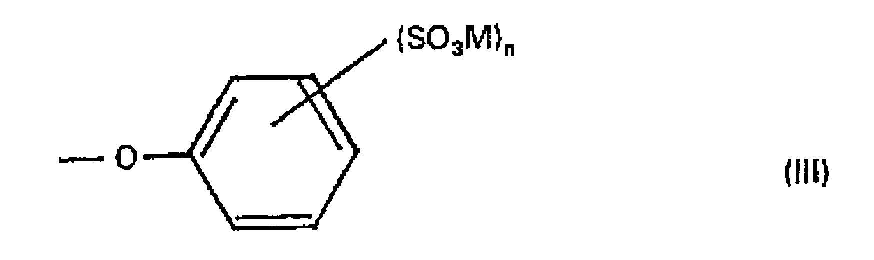 dans laquelle M est un cation et n est un nombre entier de 1 à 3.

14. Composés selon la revendication 13, **caractérisés en ce qu'**au moins un groupe parmi R³, R⁴, R⁵ et R⁶ présente la formule structurale générale (IV) : 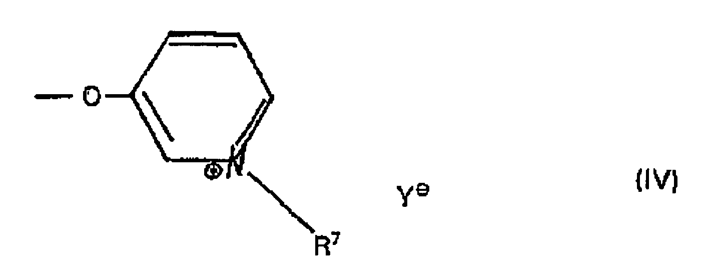 dans laquelle R⁷ représente un reste alkyle en C₁ à C₆ substitué le cas échéant et Y est un anion.

15. Conjugué de composés selon l'une quelconque des revendications 1 à 14 avec un partenaire de liaison.

16. Conjugué selon la revendication 15, **caractérisé en ce que** le partenaire de liaison est une biomolécule.

17. Procédé de préparation de bisimides de l'acide pérylène tétracarboxylique de formule structurale générale (I), comprenant les étapes consistant à :
(a) faire réagir des bisimides de l'acide pérylène di- ou tétracarboxylique substitués par des halogènes de formule structurale générale (V) : 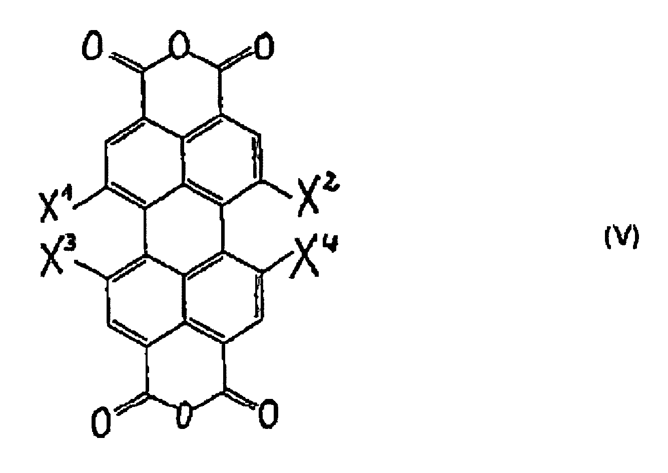 dans laquelle au moins deux groupes parmi X¹, X², X³ et X⁴ représente un halogène, en particulier Cl ou Br, et les autres un hydrogène,
avec un mélange de deux amines différentes H₂NR¹ et H₂NR², dans lesquelles R¹ et R₂ sont définis comme dans la revendication 1,
en un composé de formule structurale générale (VI) 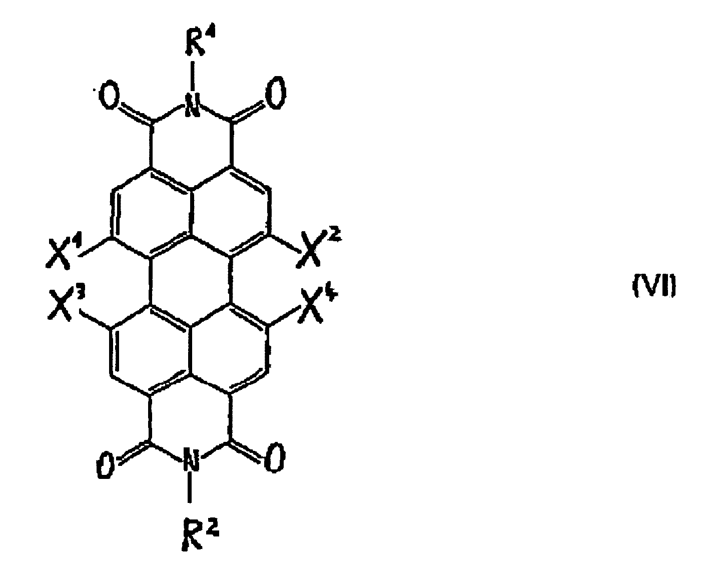
(b) faire réagir le composé (VI) avec un composé de formule structurale générale (VII)
HO-Ar' (VII)
dans laquelle Ar' représente un reste aromatique ou hétéroaromatique,
en bisimides de l'acide pérylène tétracarboxylique tétrasubstitués de formule structurale générale (VIIIa) ou en bisimides de l'acide pérylène tétracarboxylique disubstitués de formule structurale générale (VIIIb) : 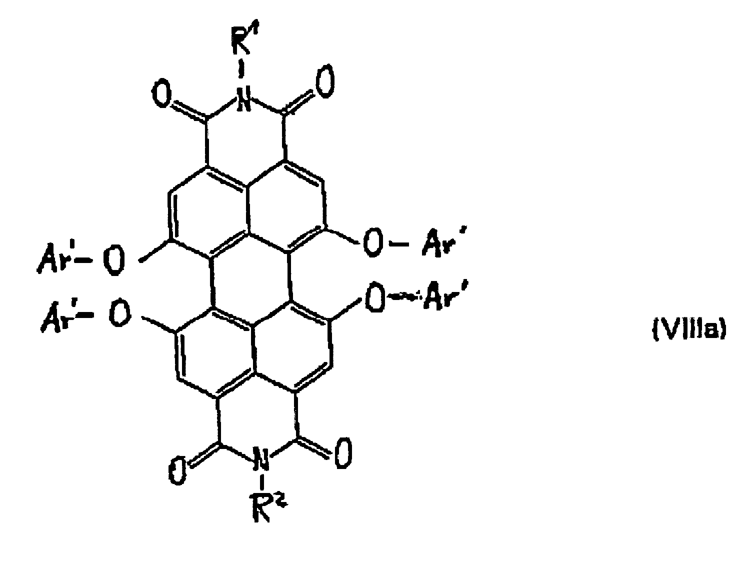 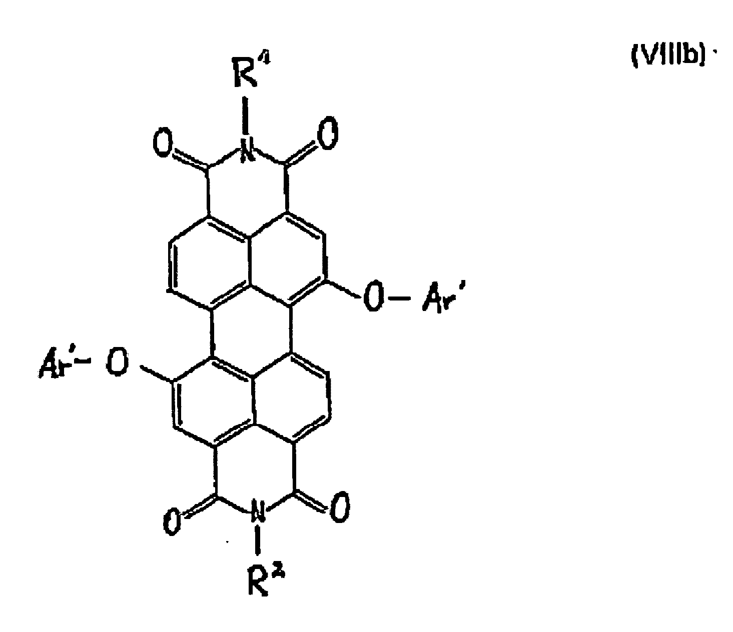 et
(c) introduire des groupes hydrophiles dans les restes aromatiques ou hétéroaromatiques Ar'.

18. Procédé selon la revendication 17, **caractérisé en ce que** l'étape (b) comprend l'introduction d'au moins un groupe SO₃H ou SO₃M dans Ar', sachant que M est un cation.

19. Procédé selon la revendication 17, **caractérisé en ce que** l'étape (b) comprend l'alkylation d'au moins un atome N hétéroaromatique dans Ar'.
